# EUROPEAN PATENT SPECIFICATION

(11) **EP 3 020 379 B1**
(45) Date of publication and mention of the grant of the patent: **27.09.2017**
(21) Application number: 15425098.9
(22) Date of filing: 16.11.2015
(51) Int. Cl.: A61F 5/56

(54) **GLOTTO-MANDIBULAR ARCHES DEVICE FOR TMJ DISEASE**
GLOTTIS-MANDIBULARBOGENVORRICHTUNG FÜR KRANIOMANDIBULÄRE DYSFUNKTION
DISPOSITIF À ARCADES GLOTTO-MANDIBULAIRE POUR MALADIE TMJ

(30) Priority: 17.11.2014 IT RM20140673
(43) Date of publication of application: 18.05.2016
(73) Proprietor: RAMPELLO, Alessandro, 00159 Roma (IT)
(72) Inventor: RAMPELLO, Alessandro, 00159 Roma (IT)
(74) Representative: Santi, Filippo

(56) References cited:
- WO-A1-2010/035303
- WO-A1-2014/018105
- US-A- 4 304 227
- US-A1- 2008 295 850
- US-A1- 2012 103 345

## Description

### FIELD

The present disclosure relates to glotto-mandibular arches devices for the relaxation and rebalancing of the muscles and skin tissues of the face and for the therapy of TMJ dysfunction and skull-cervico-mandibular disorders.

### BACKGROUND

At the international level, countless universal devices to be placed in the mouth have been proposed and all are somewhat structured to intervene only in the dental arches. To date, in fact, the Inventor does not know a universal device to be inserted in the mouth, which would also be adapted to re-educate the tongue position and to harmonize all the components of the stomatognathic and cervical spine. Positional rehabilitation of the tongue has, in fact, beneficial effects on bruxing and clenching of the teeth, caused by stress or dysfunctions, and in a reflected way prevents and treats joints disorders of the TMJ ("Temporo-Mandibular Joint") and the postural disorders of the various metameres of the spine.

Document WO2010/035303 of Rampello, which forms the basis of the preamble of claim 1, discloses a device that spaces away the teeth in a passive way not to make them come into contact, without changing the lengths of the muscles and the skull-mandibular levers. It has no rehabilitative function and therefore does not cure the underlying problem of bruxing.

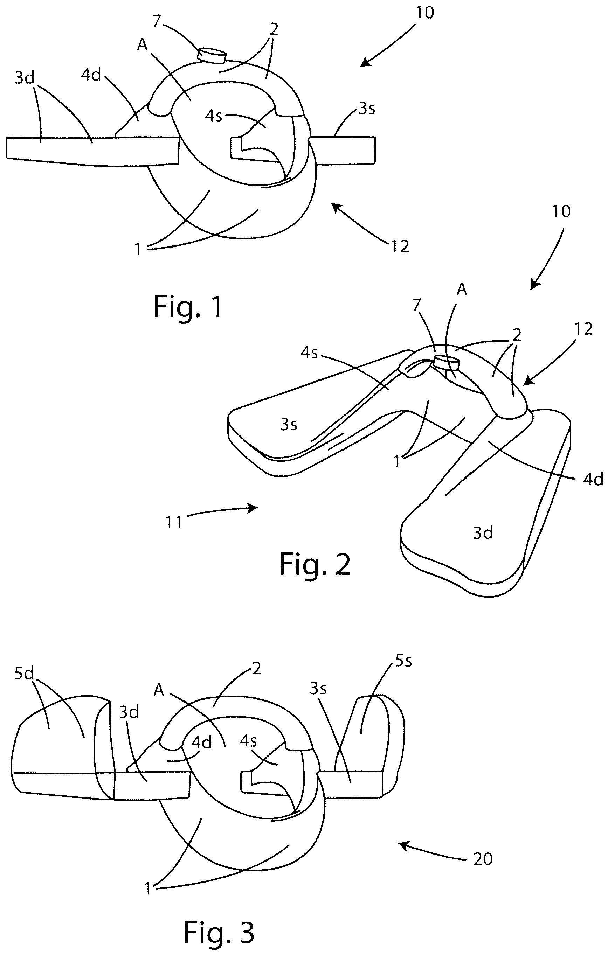

Document US 4304227 A of Samelson et al. discloses a device that forces the tongue to enter inside a cylinder 24 (Figures 2 and 3) and this tends to obstruct the mouth breathing to promote nasal breathing. This device is used basically for night snoring and its structure also provides a portion external to the mouth and lips (10 and 12 in Fig 1). Again, it does not act on the cause of bruxism, changing the lengths of the muscles and craniomandibular levers, but spaces apart only the teeth and therefore plays only a protection action against wear of the teeth and an action to improve the night snoring.

Document US2012/103345 of Gay at al. discloses a device requiring the tongue to remain low under a bridge 4 (Fig 1) to leave a free space above the tongue in order to make air pass through during breathing. Again, this device is basically used for night snoring and sleep apnea. It does not act on bruxism, changing craniomandibular levers and lengths of muscles, but spaces away the teeth and plays an opposition action and protection action aiming more to improve the night snoring.

Other general reference devices are those disclosed in documents US 2008/295850 A1, WO2014/018105.

The object of the present invention is therefore to provide a device with innovative elements, which allow a new vision of therapeutic approach and harmonized rebalancing of all the components of the stomatognathic system, basically proposing a functional positional re-education stimulus, even by a different orientation of the load vectors of muscle forces.

### BRIEF DESCRIPTION OF THE DRAWINGS

The invention will now be described by way of illustration but not by way of limitation, with particular reference to the drawings of the accompanying figures, in which:
- Fig. 1 shows a front perspective view rotated by 3/4 of a first device according to an aspect of the invention;
- Fig. 2 shows a postero-superior perspective view, rotated by 3/4, of the device according to figure 1;
- Fig. 3 shows a front perspective view rotated by 3/4 of a device according to an aspect of the invention, with the two symmetrical vertical side genial shields "5d" and "5s";
- Fig. 4 shows a front perspective view rotated by 3/4 of the device according to the invention with two symmetrical vertical side shields "5d" and "5s" and a vestibular band "6";
- Fig. 5 shows a postero-superior perspective view, rotated by 3/4, of the device according to an aspect of the invention with two symmetrical vertical side genial shields "5d" and "5s", a vestibular band "6" and a circular button "7", as well as two main planes of symmetry;
- Fig. 6 shows a postero-superior perspective view of the device according to the invention in a custom variation with ball hooks "8d" and "8s" as retention elements instead of the genial side shields "5d" and "5s" ;
- Fig. 7 shows a postero-superior perspective view rotated by 3/4 of the device according to an aspect of the invention with two symmetrical genial shields "5d" and "5s", a vestibular band "6", a circular button "7" and with reduced occlusal plans "3d" and "3s";
- Fig. 8 shows a bottom view of the device of the invention in a variation with a plate "9", which is personalized on plaster casts and with the lingual ring that is structured as modular element to be connected to and disconnected from said plate;
- Fig. 9 shows a postero-superior view of the device according to an aspect of the invention in a custom variation with extended plate with higher wings "4d" and "4s", external vertical genial shields "5d" and "5s" extended in continuation with wider front band "6";
- Fig. 10 shows a side perspective view rotated by 3/4 of the device according to an aspect of the invention in a custom variation with extended plate having higher wings "4d" and "4s", external vertical genial shields "5d" and "5s" which are extended without solution of continuity to a wider front band "6";
- Figure 11 shows the device of Figure 5 as seen from a side, parallel to the sagittal plane;
- Figure 12 shows the device of Figure 5 in the sagittal section; and
- Figure 13 shows the device of Figure 5 as seen from above.

### DETAILED DESCRIPTION

The present invention relates to a device in the form of a ring which surrounds the tongue and is joined to various structural and functional surfaces.

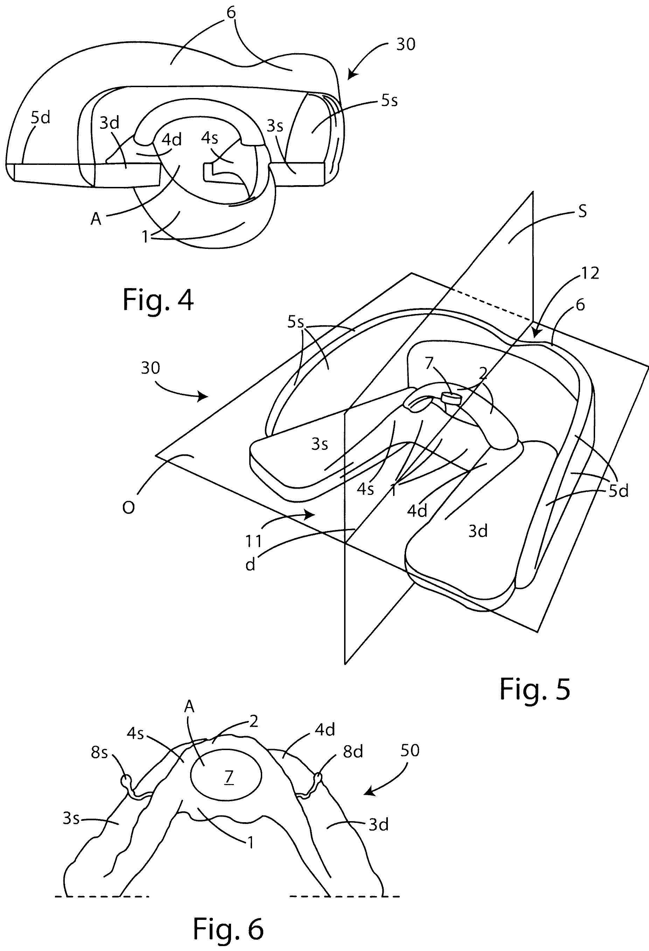

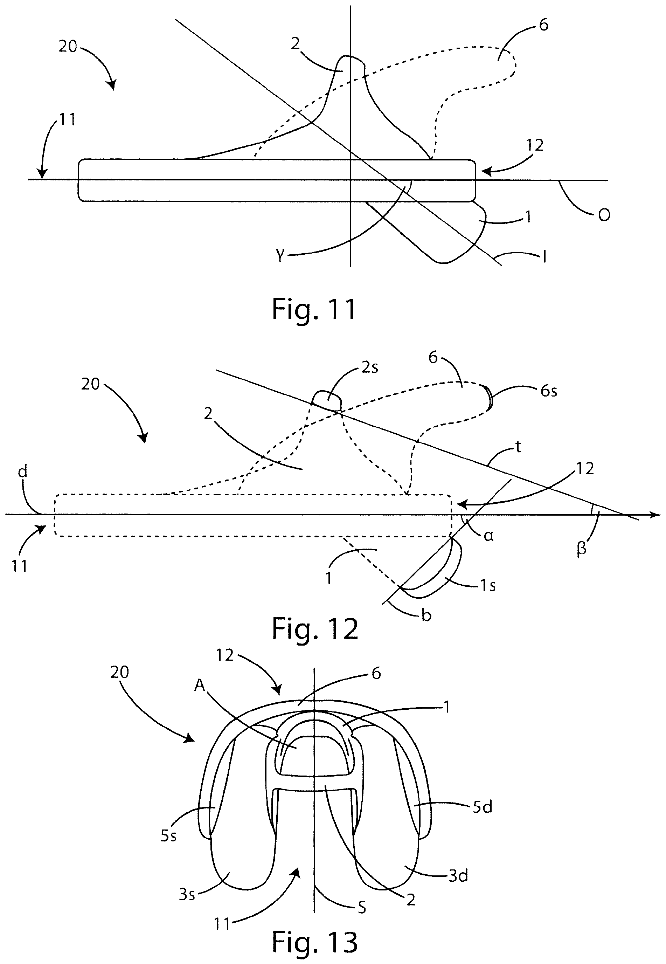

Referring to the figures, the lingual ring is formed by two arcs: the lower arc "1" and the upper arch "2", which are connected to a left surface 3s and a right surface 3d (which in this representation are trapezoidal with symmetrical left and right rounded corners), which lie on a single horizontal plane O, as shown in Figures 5, 11 (this feature may be common to all the aspects of the invention). In the following, we will always refer to an only one lower arc and only one upper arc, but it is to be understood that the arcs may be, in both cases, multiple. The arches 1, 2 and the surfaces 3s, 3d form an opening in which the tongue is to be inserted.

Referring now to Figure 5, it is seen that there is a plane of symmetry S, which in use coincides with the sagittal plane of the body, which is perpendicular to said horizontal plane O. The intersection of these two planes is a line d that defines also a direction of extension of the invention device. The two surfaces 3s and 3d are mirror-like (specular) surfaces with respect to the plane S. In general, the other parts of the device are mirror-like with respect to this plane of symmetry, as well.

The device according to the invention has a front side 12 and a rear side 11. The rear side 11 is the one which in use is positioned in the back of the mouth, whereas the front side 12 is the one which in use is positioned in front of the incisors. By this, the direction of extension d as proceeding from the rear side 11 to the front side 12 is also defined. This direction is clearly indicated in Figure 12 and is common to all the devices according to the various aspects of the invention.

Referring now to Figures 11 to 13, the positioning of the upper and lower arches is illustrated. From Figure 11, it is seen that the upper arc 2 extends above the horizontal plane O, i.e., in use, towards the palate. On the contrary, the lower arc 1 extends below the horizontal plane O, i.e., in use, towards the floor of the mouth. The lower arc 1 is inclined with respect to the horizontal plane O. Indeed, it lies on an inclined plane I which form, along the direction d, an angle γ with the horizontal plane O, which is an acute angle, i.e. less than 90°.

The upper arch 2 can be inclined in various ways or be substantially perpendicular to the horizontal plane O. What is important is that the external and internal surface of said upper arc are inclined in an appropriate manner. More in detail, with reference to Figure 12, it is seen that the inner surface of the section 2s of the upper arch 2 on the sagittal plane S has an inclination in the direction of extension d equal to an angle β that is an acute angle. The upper arch 2 is shown as palatal or retro-incisal arch, and leads to the positional re-education of the tongue-bone-hyoid complex by constituting a reference point. It extends so as to surround the palate, or behind the upper front tooth group.

Similarly, the inner surface of the section 1s on the sagittal plane S of the lower arch 1 is inclined with respect to said direction d by an acute angle α. This leads to the three-dimensional positional re-education of the whole tongue-hyoid-bone, peripharyngeal muscles, jaw and condyles complex. The arc extends downwardly in the front vestibule of the floor of the mouth and obliges the tongue to assume a higher position, it obliges the lower incisors and the lower jaw in a posture more advanced than the usual one and, as a consequence, it compels condyles, masticatory and back-of-head muscles, in a protruded position.

In this way, the lingual ring has an overall internal shape that is funnel-shaped, so that the tongue is pushed upwards (palate) with major consequences for the cure of certain disorders.

Therefore, the device with the characteristics just described, by the lingual ring, basically aims at the positional rehabilitation of the tongue and of all the components of the stomatognathic apparatus. This differentiates it from other universal devices of the prior art, which instead tend to space away the only dental arches. The re-education of the tongue has in fact beneficial effects on all components of the stomatognathic system and spine in order to facilitate the resolution of symptoms and occlusal-joint dysfunctions, such as: cervical, headaches, soreness of the column, the clenching-grinding of the teeth and dysfunctions of jaw joints, with solutions of orthodontic, speech, night snoring, sleep apnoea, tropism of mimic muscles and skin face tissues problems.

In particular, the device according to the invention is a positional rehabilitator harmonizing the active rachis and not a simple passive bearing which spaces away the teeth. It is a truly universal functional device that rehabilitates the position and the posture of all the components of the stomatognathic apparatus: tongue, hyoid bone, jaw, condyles, masticatory muscles, peripharyngeal muscles, mimic and facial skull-cervico-mandibular muscles.

These effects are maximized in the specific aspect of the invention as described in the following.

In Figures 1 and 2, it is noted that the device 10 according to the invention is substantially in the basic configuration described above. An additional feature is the shapings 4s, 4d (vertical reinforcement edges) which extend from the surfaces 3s, 3d upwards and possibly downwards and are connected to the arcs 1 and 2. This is done to better accompany the tongue in its insertion. A second optional additional feature is constituted by at least one functional rehabilitation element 7, which can have the shape of a button or other forms (for example balls, spheres, rings) adapted to the size and morphology of the oral cavity as a function also of the rehabilitative action to be performed. The elements 7 may be placed at the middle of the upper arch 2, and may serve the functional rehabilitation of the tongue: speech, phoniatric, physio-, orthodontic therapy. They may have variable shape, design, location and angle, for example they can be balls, spheres, rings, holes, grids, shields, plans, buttons, rods, bands, strings, elastics, nets, guides, depending on the size and morphology of the oral cavity and the re-educational function to be performed.

In Figure 3, a device 20 which is substantially based on the device of Figure 2 is illustrated, wherein the so-called genial walls have been added on the left 5s and on the right 5d (in the specific re-presentation they have oval shape and are mirror-like with respect to the plane S). These walls are nothing else than surfaces extending perpendicularly to the surfaces 3s, 3d upwards. They are adapted to contact the inside cheeks, and to balance the forces of the buccinatory muscles for better positioning the device according to the invention.

In Figures 4 and 5, a device 30 which is substantially based on the device of Figure 3 is illustrated, in which the two genial surfaces 5d, 5s are connected upwards and towards the front side by a connecting front band 6, which in use will be placed so as to face the front dental elements, and in the front vestibular arches (between the upper teeth and upper lip).

Figure 6 illustrates a device 50 according to an aspect of the invention wherein there are no more genial surfaces, but in their place, on the surfaces 3s, 3d, elements "8d" on the right and "8s" on the left have been fixed, which perform a function similar to that of the genial surfaces. These elements are plans, buttons, rods, guides, ball hooks, Adams clasps or orthodontic wires according to the size and morphology of the oral cavity, i.e. as a function of the action to be performed.

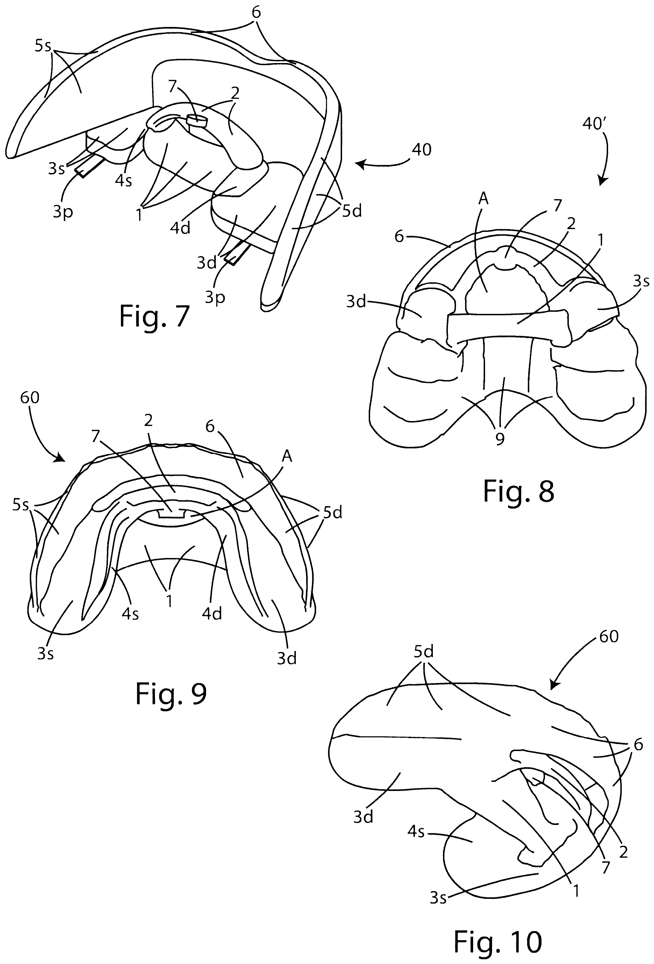

Figure 7 shows a device 40 according to an aspect of the invention, that has the structure similar to that of Figures 4 and 5, in which, however, the surfaces 3s, 3d are of reduced size. This is because in such a way the device 40 is utilizable in the assembly 40' of Figure 8. In fact, it is simply to be introduced into a palatal plate 9 by engagement means which in Figure 7 are represented by appropriate pins 3p.

Figures 9 and 10 show, according to an aspect of the invention, a device 60 substantially similar to that of Figures 4 and 5, in which, however, it is clear that the overall device is made in a single shaped piece. In fact, also the other exemplary devices can be made in a single piece, or in several pieces, according to convenience of production or use.

The different parts of the device according to the present invention may be such that the whole assembly or device is adapted to be built or printed in a standard mono-block made of various materials: plastic and/or non-toxic silicone, or realized in an individual way on models and plaster casts determined on the individual patient by the use of dental resins, with shapes, sizes, thickness, design, which can vary in relation to the size of the arches, the function, the dental class (I, II, III of Angle), or skeletal class, the age of subjects and the maxillofacial development or growth, or alternatively can be constituted by several parts of the same material which can be assembled, or, if useful for therapeutic purposes, of different materials.

In summary, the oral device 10, 20, 30, 40, 40', 50, 60 according to the invention comprises first of all a first interocclusal surface 3s that can be inserted between the dental arches on the left and a second interocclusal surface 3d insertable between the dental arches on the right. These first and second interocclusal surfaces 3s, 3d are arranged substantially on a horizontal plane O and are mirror-like with respect to a sagittal plane S perpendicular to said horizontal plane O and passing between the two interocclusal surfaces 3s, 3d.

The oral device 10 extends along an extension direction d which is formed by the intersection between said horizontal plane O and said sagittal plane S and which goes from the back side 11 positioned in use between the molars of the dental arches to the front side 12 which in use is facing the canines and incisors.

The first interocclusal surface 3s and the second interocclusal surface 3d are joined by at least one upper arc 2 which in use extends above the horizontal plane O toward the palate and by at least one lower arc 1 which in use extends below the horizontal plane O towards the floor of the mouth. The at least one lower arc 1 and the at least one upper arc 2 form an opening A dimensioned in such a way to receive in use the tongue at least partially.

Some features that actively contribute to the effect of the therapeutic device according to the invention relate to the inclination of the external and internal surface of said ring.

In particular, the at least one lower arc 1 lies on a plane I which forms with said horizontal plane O in the extension direction an acute angle γ (very interesting results have been found between 30 and 60° or between 35 and 45°), in such a way that the lower arc 1 extends towards said front side 12.

The lying plane of the lower arch can be drawn internally to said lower arc (i.e. touching the line that faces the horizontal plane) or along the side edges. The important thing is that it is always inclined with respect to the plane of the surfaces that can be inserted between the dental arches. This holds also for the lying plane of the upper arch.

Furthermore, the at least one lower arc 1 has a lower arc section 1s on the sagittal plane S inclined so as to form an acute angle α (very interesting results have been found between 30 and 55°, and between 35 and 45°) with said direction of extension d.

Finally, the at least one upper arc 2 has an upper arc section 2s on the sagittal plane S inclined so as to form an acute angle β (particularly interesting results have been found between 25 and 45°, in particular between 30 and 40°) with said direction of extension d.

These angles can be calculated in various ways, preferably with respect to the line of the section that faces the horizontal plane O. In fact, it is the contact with the line that forces the tongue to rise. These section lines facing the horizontal plane can be straight or curved, but overall they should show an inclination in order to push the tongue towards the palate.

Consequently, the tongue, which in use comes into the aperture A, is pushed towards the palate and obliges lower incisors and jaw in a posture more advanced than they would have without the oral device, causing a change in the force vectors of masticatory muscles, in particular for the treatment of aforementioned skull-cervico-mandibular dysfunctions and annexed tissues.

The above-described device can be further improved with respect to the action of the buccinatory muscles, building it in such a way that on an edge of said first interocclusal surface 3s that, in use, faces a genial wall, a first genial surface 5s is connected which is substantially perpendicular to said first interocclusal surface 3s, so that, on an edge of said second interocclusal surface 3d that, in use, faces a genial wall, a second genial surface 5d is connected which is substantially perpendicular to said second interocclusal surface 3d. In such a way, said first surface 5s and said second genial surface 5d are arranged, in use, between the upper dental arch and the respective genial surfaces. Between said first 5s and said second genial surface 5d, a vestibular band 6 can be connected which runs substantially on said front side 12 and is adapted to be placed, in use, between the upper incisors and upper lip.

Although the device according to the invention is autonomous, it can be inserted in a palate plate 9 realized starting from individual plaster casts.

To be able to decline further therapeutical properties of the device of the invention, one or more functional rehabilitative elements 7 are arranged in the vicinity of the top of said at least one upper arc 2, for examples buttons or rotating spheres.

In place of the genial surfaces, the device may have at least a first retaining element 8s fixed to said first surface 3s and at least one second retaining element 8d fixed to said second surface 3d.

For a better ergonomics of the device and its better positioning in the mouth, said first interocclusal surface 3s and said second interocclusal surface 3d have appropriate respective shapings 4s, 4d on the side facing the center of the mouth.

The device according to the invention can thus assume an oblong shape (in extension direction d) or similar to a horseshoe with the ring that surrounds the soft parts of the tongue and the horizontal planes, the shields, and the front band that embrace other soft tissue and the teeth, in order to perform the stabilization function and allow the re-education and the positional disengagement of the teeth.

The present invention provides a device in the form of an irregular, funnel-shaped ring that surrounds the tongue. According to an aspect of the invention, the device can be conveniently be constituted by three parts: A) a central part or the real lingual ring; B) an optional part of the systems of reinforcement, anchoring, balancing, connection and stabilization; C) an optional part of the rehabilitative functional reference systems. The device of the invention is a modifier of the vectors of the forces of the chewing muscles, that provides an active positional re-education of the anatomical and functional structures of the skull-cervical-mandibular unit and does not constitute a simple passive bearing which spaces apart the teeth, but a real universal functional device that re-educates the position and posture of all the components of the stomatognathic system: tongue, hyoid bone, jaw, condyles, masticatory muscles, peri-pharyngeal muscles, mimic facial muscles with associated skin tissues and skull-cervical-mandibular tissues.

In the foregoing, aspects of the invention have been described and variations of the invention have been suggested, but it is to be understood that those skilled in the art can make other variations and changes, without so departing from the related scope of protection, as defined by the appended claims.

## Claims

1. Oral device (10, 20, 30, 40, 40', 50, 60), comprising:
a first interocclusal surface (3s) insertable between the left tooth arches,
a second interocclusal surface (3d) insertable between the right tooth arches,
said first and second interocclusal surfaces (3s, 3d) being disposed substantially on a horizontal plans (O) and being specular with respect to a sagittal plane (S) perpendicular to said horizontal plane (O) and passing between the two interocctusal surfaces (3s, 3d),
the oral device (10, 20, 30, 40, 40', 50, 60) extending along an extension direction (d) that is formed by the intersection between said horizontal plane (O) and said sagittal plane (S) and goes from the back side (11), that can be positioned in use between molar teeth of the tooth arches, to the front side (12), which in use is facing the canine teeth and incisors,
wherein:
said first interocclusal surface (3s) and said second interocclusal surface (3d) are united by at least an upper arch (2), which in use extends above said horizontal plane (O) towards the palate, and at least a lower arch (1), which in use extends below said horizontal plane (O) towards the mouth floor;
said at least a lower arch (1) and said at least an upper arch (2) forming an aperture (A) dimensioned in such a way to receive in use at least partially the tongue; **characterised in that**
said at least a lower arch (1) lies on a plane (l) forming with said horizontal plane (O) in said extension direction (d) an acute angle γ, in such a way that the lower arch (1) extends towards said front side (12);
said at least a lower arch (1) has a lower arch section (1s) on the sagittal plane (S) inclined in such a way to form an acute angle α with said extension direction (d);
said at least un upper arch (2) has an upper arch section (2s), on the sagittal plane (S), inclined in such a way to form an acute angle β with said extension direction (d); and
so that, as a consequence, the tongue that in use enters said aperture (A) is pushed towards the palate and compels the lower incisors and the mandible in a posture that is more advanced with respect to the posture without the oral device (10, 20, 30, 40, 40', 50, 60), causing a variation of vectors of forces of the masticatory muscles, in particular for the treatment of said skull-cervico-mandibular and relevant tissues dysfunctions.

2. Oral device according to claim 1, wherein said angle γ is comprised between 30 and 60°.

3. Oral device according to claim 2, wherein said angle γ is comprised between 35 and 45°.

4. Oral device according to any claim 1 to 3, wherein said acute angle α is comprised between 30 and 55°, in particular between 35 and 45°.

5. Oral device according to any claim 1 to 4, wherein said acute angle β is comprised between 25 and 45°, in particular between 30 and 40°.

6. Oral device (20) according to any claim 1 to 5, wherein on an edge of said first interocclusal surface (3s) that, in use, faces a genial wall, a first genial surface (5s) is connected, which is substantially perpendicular to said first interocclusal surface (3s), and in that on the edge of said second interocclusal surface (3d) that, in use, faces a genial wall, a second genial surface (5d) is connected, which is substantially perpendicular to said second interocclusal surface (3d), in such a way that said first genial surface (5s) and said second genial surface (5d) are disposed, in use, between the upper tooth arch and the respective genial surfaces.

7. Oral device (30) according to claim 6, wherein between said first and said second genial surface (5s, 5d) a vestibular band (6) is connected, which runs substantially on said front side and is adapted to dispose, in use, between the upper incisors and the upper lip.

8. Oral device (40, 40') according to any claim 1 to 7, wherein said oral device is insertable in a palate plate (9) realized starting from individual plaster cast.

9. Oral device according to any claim 1 to 8, wherein close to the top of said at least an upper arch (2), the oral device comprises one or more functional re-educative elements (2), in particular buttons or rotating spheres.

10. Oral device (50) according to any claim 1 to 9, when not depending on claim 6, wherein it comprises at least a first retention element (8s) fixed to said first surface (3s) and at least a second retention element (8d) fixed to said second surface (3d).

11. Oral device according to any claim 1 to 10, wherein said first interocclusal surface (3s) and said second interocclusal surface (3d) present suitable respective shapings (4s, 4d) on a side facing the centre of the mouth.

12. Oral device according to any claim 1 to 11, wherein it is formed in a single piece.

## Patentansprüche

1. Orale Vorrichtung (10, 20, 30, 40, 40', 50, 60), umfassend:
eine erste interokklusale Oberfläche (3s), einsetzbar zwischen die linken Zahnbögen,
eine zweite interokklusale Oberfläche (3d), einsetzbar zwischen die rechten Zahnbögen,
wobei die ersten und zweiten interokklusalen Oberflächen (3s, 3d) im Wesentlichen auf einer horizontalen Ebene (O) angeordnet sind und hinsichtlich einer Sagittalebene (S) rechtwinklig zu der horizontalen Ebene (O) spekular sind und zwischen den zwei interokklusalen Oberflächen (3s, 3d) hindurchführen, wobei
die orale Vorrichtung (10, 20, 30, 40, 40', 50, 60) sich entlang einer Ausdehnungsrichtung (d) erstreckt, die durch den Schnittpunkt zwischen der horizontalen Ebene (O) und der Sagittalebene (S) gebildet wird und von der Rückseite (11),
die bei Gebrauch zwischen molaren Zähnen der Zahnbögen positioniert sein kann, zu der Vorderseite (12) verläuft, welche bei Gebrauch zu den Eckzähnen und Schneidezähnen weist,
wobei:
die erste interokklusale Oberfläche (3s) und die zweite interokklusale Oberfläche vereinigt sind (3d) durch mindestens einen oberen Bogen (2), welcher sich bei Gebrauch über die horizontale Ebene (O) zu dem Gaumen erstreckt, und mindestens einen unteren Bogen (1), welcher sich bei Gebrauch unter die horizontale Ebene (O) zu dem Mundboden erstreckt;
wobei der mindestens eine untere Bogen (1) und der mindestens eine obere Bogen (2) eine Öffnung (A) in einer derartigen Weise bemessen ausbilden, um bei Gebrauch mindestens teilweise die Zunge aufzunehmen;
**dadurch gekennzeichnet, dass**
der mindestens eine untere Bogen (1) auf einer Ebene (I) liegt, die mit der horizontalen Ebene (O) in der Ausdehnungsrichtung (d) einen spitzem Winkel γ in einer derartigen Weise bildet, dass der untere Bogen (1) sich zu der Vorderseite (12) erstreckt;
der mindestens eine untere Bogen (1) einen unteren Bogenabschnitt (1s) auf der Sagittalebene (S) aufweist, in einer derartigen Weise geneigt, dass sich ein spitzer Winkel α mit der Ausdehnungsrichtung (d) bildet;
der mindestens eine obere Bogen (2) einen oberen Bogenabschnitt (2s) auf der Sagittalebene (S) aufweist, in einer derartigen Weise geneigt, dass sich ein spitzer Winkel ß mit der Ausdehnungsrichtung (d) bildet; und
so, dass als eine Folge die Zunge, die bei Gebrauch in die Öffnung (A) eindringt, zu dem Gaumen geschoben wird und die unteren Schneidezähne und die Mandibula in eine Haltung zwingt, die hinsichtlich der Haltung ohne die orale Vorrichtung (10, 20, 30, 40, 40', 50, 60) mehr nach vorn ist,
was eine Variation von Kraftvektoren der mastikatorischen Muskeln veranlasst, insbesondere für die Behandlung der Schädel-Zerviko-mandibulären und relevanten Gewebe-Dysfunktionen.

2. Orale Vorrichtung nach Anspruch 1, wobei der Winkel γ zwischen 30 und 60° ausgebildet ist.

3. Orale Vorrichtung nach Anspruch 2, wobei der Winkel γ zwischen 35 und 45° ausgebildet ist.

4. Orale Vorrichtung nach einem Anspruch 1 bis 3, wobei der spitze Winkel α zwischen 30 und 55°, insbesondere zwischen 35 und 45°, ausgebildet ist.

5. Orale Vorrichtung nach einem Anspruch 1 bis 4, wobei der spitze Winkel ß zwischen 25 und 45°, insbesondere zwischen 30 und 40°, ausgebildet ist.

6. Orale Vorrichtung (20) nach einem Anspruch 1 bis 5, wobei mit einer Kante der ersten interokklusalen Oberfläche (3s), die bei Gebrauch zu einer Kinnwange weist, eine erste kinnseitige Fläche (5s) verbunden ist, die im Wesentlichen rechtwinklig zu der ersten interokklusalen Oberfläche (3s) ist, und wobei mit der Kante der zweiten interokklusalen Oberfläche (3d), die bei Gebrauch zu einer Kinnwange weist, eine zweite kinnseitige Fläche (5d) verbunden ist, die im Wesentlichen rechtwinklig zu der zweiten interokklusalen Oberfläche (3d) ist, in einer derartigen Weise, dass die erste kinnseitige Fläche (5s) und die zweite kinnseitige Fläche (5d) bei Gebrauch zwischen dem oberen Zahnbogen und den jeweiligen kinnseitige Flächen angeordnet ist.

7. Orale Vorrichtung (30) nach Anspruch 6, wobei zwischen der ersten und der zweiten kinnseitige Fläche (5s, 5d) ein vestibuläres Band (6) befestigt ist, das im Wesentlichen auf der Vorderseite verläuft und angepasst ist, um bei Gebrauch zwischen den oberen Schneidezähnen und der oberen Lippe angeordnet zu werden.

8. Orale Vorrichtung (40, 40') nach einem Anspruch 1 bis 7, wobei die orale Vorrichtung in eine Gaumenplatte (9) einsetzbar ist, realisiert, ausgehend von einem individuellen Gipsabdruck.

9. Orale Vorrichtung nach einem Anspruch 1 bis 8, wobei nahe zu dem Oberen des mindestens einen oberen Bogens (2) die orale Vorrichtung ein oder mehrere funktionelle reedukative Elemente (2), insbesondere Knöpfe oder rotierende Kugeln, umfasst.

10. Orale Vorrichtung (50) nach einem Anspruch 1 bis 9, wenn nicht von Anspruch 6 abhängig, wobei sie mindestens ein erstes Retentionselement (8s), befestigt an der ersten Oberfläche (3s), und mindestens ein zweites Retentionselement (8d), befestigt an der zweiten Oberfläche (3d), umfasst.

11. Orale Vorrichtung nach einem Anspruch 1 bis 10, wobei die erste interokklusale Oberfläche (3s) und die zweite interokklusale Oberfläche (3d) geeignete jeweilige Formen (4s, 4d) auf einer Seite, die zu der Mitte des Mundes weist, darstellen.

12. Orale Vorrichtung nach einem Anspruch 1 bis 11, wobei sie als ein einziges Stück geformt ist.

## Revendications

1. Dispositif oral (10, 20, 30, 40, 40', 50, 60), comprenant :
une première surface interocclusale (3s) insérable entre les arcs dentaires gauche, une deuxième surface interocclusale (3d) insérable entre les arcs dentaires droit,
lesdites première et deuxième surfaces interocclusales (3s, 3d) étant disposées sensiblement sur un plan horizontal (O) et étant spéculaires par rapport à un plan sagittal (S) perpendiculaire audit plan horizontal (O) et passant entre les deux surfaces interocclusales (3s, 3d),
le dispositif oral (10, 20, 30, 40, 40', 50, 60) s'étendant le long d'une direction d'extension (d) qui est formée par l'intersection entre ledit plan horizontal (O) et ledit plan sagittal (S) et va du côté arrière (11), qui peut être positionné en utilisation entre les dents molaires des arcs dentaires, au côté avant (12), qui en utilisation est orienté vers les dents canines et les incisives,
dans lequel :
ladite première surface interocclusale (3s) et ladite deuxième surface interocclusale (3d) sont unies par au moins un arc supérieur (2), qui en utilisation s'étend au-dessus dudit plan horizontal (O) vers le palais, et au moins un arc inférieur (1), qui en utilisation s'étend en dessous dudit plan horizontal (O) vers le plancher de la buccal ;
ledit au moins un arc inférieur (1) et ledit au moins un arc supérieur (2) formant une ouverture (A) dimensionnée de sorte à recevoir en utilisation au moins partiellement la langue ;
**caractérisé en ce que**
ledit au moins un arc inférieur (1) se trouve sur un plan (I) formant avec ledit plan horizontal (O) dans ladite direction d'extension (d) un angle aigu y, de sorte que l'arc inférieur (1) s'étend vers ledit côté avant (12) ;
ledit au moins un arc inférieur (1) a une section d'arc inférieur (1s) sur le plan sagittal (S) inclinée de sorte à former un angle aigu α avec ladite direction d'extension (d) ;
ledit au moins un arc supérieur (2) a une section d'arc supérieur (2s), sur le plan sagittal (S), inclinée de sorte à former un angle aigu β avec ladite direction d'extension (d) ; et
de sorte que, par conséquent, la langue qui en utilisation entre dans ladite ouverture (A) est poussée vers le palais et contraint les incisives inférieures et la mandibule dans une posture qui est plus avancée par rapport à la posture sans le dispositif oral (10, 20, 30, 40, 40', 50, 60),
provoquant une variation de vecteurs des forces des muscles masticateurs, en particulier pour le traitement des dysfonctionnements crânio-cervico-mandibulaires et des tissus correspondants.

2. Dispositif oral selon la revendication 1, dans lequel ledit angle γ est compris entre 30 et 60°.

3. Dispositif oral selon la revendication 2, dans lequel ledit angle γ est compris entre 35 et 45°.

4. Dispositif oral selon l'une quelconque des revendications 1 à 3, dans lequel ledit angle aigu α est compris entre 30 et 55°, en particulier entre 35 et 45°.

5. Dispositif oral selon l'une quelconque des revendications 1 à 4, dans lequel ledit angle aigu β est compris entre 25 et 45°, en particulier entre 30 et 40°.

6. Dispositif oral (20) selon l'une quelconque des revendications 1 à 5, dans lequel sur un bord de ladite première surface interocclusale (3s) qui, en utilisation, fait face à une paroi géniale, une première surface géniale (5s) est connectée, qui est sensiblement perpendiculaire à ladite première surface interocclusale (3s), et en ce que sur le bord de ladite deuxième surface interocclusale (3d) qui, en utilisation, fait face à une paroi géniale, une deuxième surface géniale (5d) est connectée, qui est sensiblement perpendiculaire à ladite deuxième surface interocclusale (3d), de sorte que ladite première surface géniale (5s) et ladite deuxième surface géniale (5d) sont disposées, en utilisation, entre l'arc dentaire supérieure et les surfaces géniales respectives.

7. Dispositif oral (30) selon la revendication 6, dans lequel entre ladite première et ladite deuxième surface géniale (5s, 5d) une bande vestibulaire (6) est connectée, qui court sensiblement sur ledit côté avant et est adaptée pour se disposer, en utilisation, entre les incisives supérieures et la lèvre supérieure.

8. Dispositif oral (40, 40') selon l'une quelconque des revendications 1 à 7, dans lequel ledit dispositif oral est insérable dans une plaque de palais (9) réalisée en commençant par un plâtre individuel.

9. Dispositif oral selon l'une quelconque des revendications 1 à 8, dans lequel à proximité du sommet dudit au moins un arc supérieur (2), le dispositif oral comprend un ou plusieurs éléments rééducatifs fonctionnels (2), en particulier des boutons ou des sphères tournantes.

10. Dispositif oral (50) selon l'une quelconque des revendications 1 à 9, lorsqu'il ne dépend pas de la revendication 6, dans lequel il comprend au moins un premier élément de rétention (8s) fixé à ladite première surface (3s) et au moins un deuxième élément de rétention (8d) fixé à ladite deuxième surface (3d).

11. Dispositif oral selon l'une quelconque des revendications 1 à 10, dans lequel ladite première surface interocclusale (3s) et ladite deuxième surface interocclusale (3d) présentent des formes respectives adaptées (4s, 4d) sur un côté orienté vers le centre de la bouche.

12. Dispositif oral selon l'une quelconque des revendications 1 à 11, dans lequel il est formé en une seule pièce.
